(19) Europäisches Patentamt
European Patent Office
Office européen des brevets

(11) Publication number: **0 079 920**
**B1**

(12) # EUROPEAN PATENT SPECIFICATION

(45) Date of publication of patent specification: **15.10.86**

(51) Int. Cl.⁴: **C 07 D 273/00,** C 07 D 273/01, C 07 D 273/08, C 07 D 413/06

(21) Application number: **82901630.2**

(22) Date of filing: **28.05.82**

(86) International application number: **PCT/NO82/00030**

(87) International publication number: **WO 82/04253 09.12.82 Gazette 82/29**

(54) **NEW AZA-CROWN-ETHERS AND THEIR USE.**

(30) Priority: **01.06.81 NO 811839**

(43) Date of publication of application: **01.06.83 Bulletin 83/22**

(45) Publication of the grant of the patent: **15.10.86 Bulletin 86/42**

(84) Designated Contracting States: **AT BE CH DE FR GB LI LU NL SE**

(56) References cited:
**DE-A-2 250 321**
**GB-A-2 024 622**
**SE-B- 383 885**
**US-A-3 847 949**
**US-A-4 001 279**

**Tetrahedron Letters No 44 (1978), (GB); PING-LIN KUO et al.: "New synthesis of N-alkyl monoaza crown ethers", pp. 4273-4276**
**Journal Chemical Society, Perkin Translations I 1973:6 (London); LOOKHART et al: "Preparation of some nitrogen-containing polyether 'Crown' compounds", pp. 577-581**
**Tetrahedron Letter No 36 (1980), (GB);**

**BRADSHAW and STOTT: "Preparation of derivatives and analogs of the macrocyclic oligomers of ethyleneoxide (cro wn compounds)", pp. 481-484**

(73) Proprietor: **BORREGAARD INDUSTRIES LIMITED**
**P.O. Box 162**
**N-1701 Sarpsborg (NO)**

(72) Inventor: **DALE, Johannes**
**Kjemisk Institutt P.B. 1033 Blindern**
**N-Oslo 3 (NO)**
Inventor: **CALVERLEY, Martin J.**
**Lovens Kemiske Fabrikk**
**DK-2750 Ballerup (DK)**

(74) Representative: **Perry, Robert Edward et al**
**GILL JENNINGS & EVERY 53-64 Chancery Lane**
**London WC2A 1HN (GB)**

(56) References cited:
**Journal of The Chemical Society, No 14 (1981), (GB); CALVERLEY M., DALE, J.: "Synthesis and cation complexing of NN-bridged bis-(1,4,7-tioxa-10-azacyclododecanes)**
**Cyclophanes I, Ed. F. Vögte, Springer Verlag 1983**

**Journal Chemical Society, Chemical Communications (1981), pp. 684-686, 1084-1086**

EP 0 079 920 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel aza-crown ethers, and to their use as complexing agents.

The term "crown ether" was originally given to cyclic oligomers of ethylene oxide, but is now also used for analogues thereof in which one or more of the oxygen atoms have been replaced by other hetero atoms. Thus, aza-crown ethers contain one or more nitrogen atoms. Several crown ethers are disclosed in, for example, Tetrahedron Letters *36* (1980) 461.

Crown ethers have good complexing properties which may be utilised in several ways, e.g. for the extraction of metal ions into organic solvents and for separation. The Tet. Lett. article given above suggests that bicyclic aza-crown ethers, also called cryptands, in which the rings are linked together through two or more nitrogen atoms in each ring, possess particularly superior complexing ability, yielding complexes called cryptates.

DE—A—2250321, Example 9, discloses the compound 1,4,10,13-tetraaza-7,16,21,26-tetraoxatricyclo-[11,5,5,$5^{4,10}$]octacosane but gives no indication as to how that compound might be prepared.

A first novel compound according to the present invention is 1,2-bis(1-aza-4,7,10-trioxa-1-cyclododecanyl)ethane which has the formula

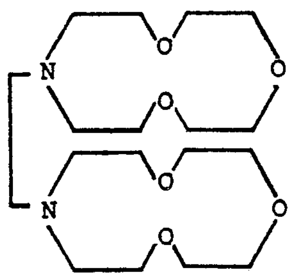

(5)

This compound may be prepared by reacting bis[2-(2-iodoethoxy)ethyl] ether (alternatively named as 1,11-diiodo-3,6,9-trioxaundecane) with 2-(1-aza-4,7,10-trioxa-1-cyclodecanyl)ethylamine or by reacting 1-aza-4,7,10-trioxacyclododecane with a suitable bridging reagent such as oxalyl chloride (followed by reduction), ethylene iodide or, preferably, ethylene ditosylate.

A second novel compound according to the present invention is 1,4,10,13-tetraaza-7,16,21,26-tetraoxa-tricyclo[11,5,5,$5^{4,10}$]octacosane which has the formula

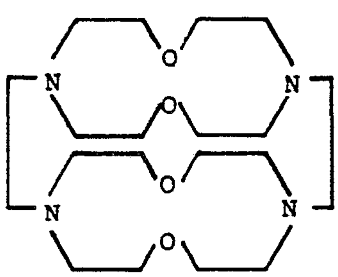

(6)

This compound may be prepared by reacting a compound of the formula

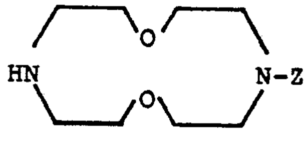

in which Z represents a protective group, with a suitable bridging agent such as ethylene ditosylate, so that two of the protected compounds are linked together by means of a bridge between the two unprotected nitrogen atoms. The protective groups are then removed, to give 1,2-bis(1,7-diaza-4,10-dioxa-1-cyclododecanyl)ethane, and a further bridge between the two rings is formed, e.g. using ethylene ditosylate again; in order to avoid the formation of polymeric material, a template cation such as lithium is used in forming this second bridge.

The novel aza-crown ethers of the invention have generally good complexing ability, depending on the ion to be complexed. Their good complexing ability with respect to stability and selectivity towards ions such as $Na^+$ and $Ca^{++}$ is related to the linking of two crown ether moieties by ethylene chains between the nitrogen atoms in the crown ether moieties. Model considerations show that complexes of "sandwich"

structure have the most favourable conformation, and will accordingly give the most stable complexes, in such cases.

For certain applications such as ion transport in lipophilic membranes, it may be suitable to anchor the molecules to larger hydrophobic groups or polymeric compounds. This anchoring should take place by attachment to one of the methylene groups in the chain or chains between the two crown ether moieties.

The following Examples illustrate the preparation of the new compounds, and the Preparations illustrate how to prepare starting materials.

Melting points (mp) were measured on a Kofler hot-stage apparatus and are uncorrected. Gas liquid chromatographic analyses were performed on a Hewlett-Packard 5700A Gas Chromatograph equipped with a flame ionisation detector. Dilute solutions in dichloromethane or the reaction solvent were injected into a 2 m × 2 mm 10% SP 2100 on Chromosorb W AW DMCS 80/100 column using nitrogen as carrier gas (flow rate 20 ml/min). The temperature of the column was initially 100°C, increasing at 16°C degree/min immediately after injection up to a maximum of 280°C. Approx. retention times ($t_P$) are quoted in minutes. Analytical thin layer chromatography (tlc) was carried out on Merck plates pre-coated with Kieselgel 60F—254. The eluant systems used were (A) a 10% solution of methanol in chloroform and (B) a 20% solution of ethyl acetate in chloroform. Elemental analyses were carried out at Mikroanalytisches Laboratorium Ilse Beetz, 8640 Kronach, West Germany. Infra-red spectra were recorded on a Jasco IRA—1 spectrophotometer and, unless stated otherwise, run as thin films between sodium chloride discs. Absorptions are given in $cm^{-1}$. Proton nuclear magnetic resonance spectra were recorded on a Varian HA 100 spectrometer and were run as solutions in deuterochloroform, except where otherwise stated, chemical shifts (δ (*H*)) being quoted in ppm relative to tetramethyl-silane (TMS) as lock and internal standard. Coupling constants (J) are given in Hz. Carbon-13 nuclear magnetic resonance spectra were recorded at 15 MHz on a JEOL JNM—FX60 Fourier Transform spectrometer, with protons noise decoupled, and run in acetonitrile solution (unless otherwise stated). Chemical shifts (δ (C)) are quoted in ppm relative to internal TMS or the solvent ($\underline{C}H_3CN$, δ 1.75; $\underline{C}H_3OH$, δ 49.90, relative to internal TMS). Assignments of particular signals to particular carbon atoms in the molecule is based upon (a) the height of the signal, which was in direct proportion to the number of equivalent atoms assigned a particular chemical shift, except in the case of quaternary carbons (which were readily identified by their proportionally lower signal heights), (b) re-running the spectrum under conditions of proton off-resonance (only in useful cases), and (c) self-consistent analogy between the spectra of the crowns studied. Mass spectra were recorded with a Micromass 7070F spectrometer. Electron impact mass spectra (70 eV) were run after direct insertion of the sample into the source maintained at *ca* 230°C. Only readily assigned *m/e* (rel. intensity%) values and the base peak are quoted. Chemical ionisation mass spectra gave a quasi-molecular ion $(M+1)^+$ as the only significant signal and were obtained using isobutane as ionisation gas.

Analytical grade reagents and solvents were used where available throughout the following preparations. Acetonitrile and N,N-dimethylformamide were dried over molecular sieves. Dichloromethane was purified by passing through a column of alumina and then distilled from calcium hydride. Pyridine and tetrahydrofuran were dried by distillation from calcium hydride and lithium aluminium hydride, respectively, immediately before use.

### Preparation 1

Bis-[2-(2-chloroethoxy)ethyl]ethyl (1)

Thionyl chloride (195 g, 1.64 mol) was added to a mechanically stirred and ice-cooled solution of tetraethylene glycol (150 g, 0.77 mol) in dry pyridine (135 g, 1.71 mol) at a rate such that the temperature of the reaction mixture remained below 50°C. Stirring was then continued at room temperature overnight (during which time the initially-formed precipitate had largely redissolved) before adding water (20 ml) and extracting with ether (100 ml, ×5), adding a further 20 ml of water before each subsequent extraction. The ether layers were combined, washed with water (100 ml), brine (100 ml), dried ($MgSO_4$) and concentrated *in vacuo*. Distillation of the residue through a short Vigreux column gave the dichloride (1) (148 g, 83%) as a colourless liquid, bp 106—108°C/0.1 mm Hg, $t_R$ 8.5, δ (*H*) 3.43—3.77 (complex symmet. m, $OC\underline{H}_2C\underline{H}_2Cl$) and 3.56 (sharp s, $OC\underline{H}_2C\underline{H}_2O$), *m/e* (rel. intensity) 153 (0.6), 151 (1.3), 139 (3.3), 137 (10), 109 (13), 107 (40), 65 (32) and 63 (100).

### Preparation 2

Bis-[2-(2-iodoethoxy)ethyl]ether (2)

Powdered sodium iodide (100 g, 0.67 mol) was added to a solution of the dichloride (1) (65.0 g, 0.28 mol) in acetone (150 ml), and the stirred mixture heated under reflux for 70h. After cooling, the reaction mixture was filtered (washing with acetone) and the filtrate concentrated *in vacuo*, diluted with ethyl acetate (200 ml) and extracted with 20% sodium thiosulphate solution (50 ml). The organic layer was washed with water (100 ml), brine (100 ml) and dried ($MgSO_4$). Removal of the solvent *in vacuo* gave the diiodide (2) as a very pale yellow oil, $t_R$ 10.5. Glc showed the presence of about 2% of the chloro-iodide (2b) as the only significant impurity ($t_R$ 9.3). δ (*H*) 3.19 (4H, t, J 7, $OCH_2C\underline{H}_2I$), 3.58 (8H, s, $OC\underline{H}_2C\underline{H}_2O$) and 3.70 (4H, t, J 7, $OC\underline{H}_2CH_2I$), *m/e* 229 (1.5, 199 (7) and 155 (100).

Crystallisation from ether solution gave the diiodide (2) as needles, mp 92—93°, initially colourless but soon becoming yellow on storage.

Preparation 3

N-benzyl-1-aza-4,7,10-trioxacyclododecane (3)

A stirred solution of the diiodide (2) (30.0 g oil, equivalent to 0.073 mol dichloride (1)) and benzylamine (8.2 g, 0.77 mol) in dry acetonitrile (1 litre) containing suspended powdered anhydrous sodium carbonate (30 g) was heated under reflux under an atmosphere of nitrogen for 18 hours. The cooled solution was decanted from most of the solid and concentrated *in vacuo*. The residues from decantation and concentration were combined and partitioned between water (600 ml) and ether (300 ml). The aqueous layer was re-extracted with ether (200 ml × 2), and the combined organic layers washed with water (200 ml), brine (200 ml) and dried ($Na_2SO_4$). After removing the ether *in vacuo*, the residue was distilled through a short path, collecting material volatile at 0.05 mm Hg with a bath temperature up to 200°C. The distillate was redistilled through a short Vigreux column to give the pure aza-crown (3) (10.5 g, 54%) as a colourless oil, bp 140—143°C/0.05 mm Hg, $t_R$ 11.5.

| | | | |
|---|---|---|---|
| $C_{15}H_{23}NO_3$ (265.3) requires: | C, 67.89; | H, 8.74; | N, 5.28% |
| Found: | C, 67.75; | H, 8.68; | N, 5.20%. |

δ (*H*) 2.69 (4H, t, J 5, $OCH_2C\underline{H}_2N$), 3.55 (4H, t, J 5, $OC\underline{H}_2CH_2N$), 3.60 (10H, s, $OC\underline{H}_2C\underline{H}_2O$ and $PhC\underline{H}_2N$) and 7.02—7.30 (5H, m, Ar$\underline{H}$).

δ (*C*) 55.65 (N—C (ring)), 61.42 (N—$\underline{C}$—pH), 70.64, 70.97 and 71.75 (O—$\underline{C}$), 127.59

(C—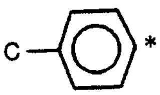*),

128.95 and 129.79 (remaining tert. arom. *c*) and 141.09 (quatern. arom. *C*), *m/e* 265 ($M^+$, 2) and 91 (100).

Preparation 4

1-Aza-4,7,10-trioxacyclododecane (4)

A 10% palladium-on-activated carbon catalyst (0.5 g) was added to a solution of the N-benzyl-aza-12-crown-4 (3) in acetic acid (10 ml) and the suspension was stirred at 60°C under 3 atmospheres of hydrogen for 15 hours. The cooled mixture was filtered through a bed of Celite, using ethanol for washing. The solution was concentrated *in vacuo* and the residue dissolved in water (10 ml). After basification with 30% potassium carbonate solution (20 ml), the solution was extracted with chloroform (25 ml × 4) and the combined organic layers were washed with 10% potassium carbonate solution (10 ml) and brine (20 ml), and dried ($Na_2SO_4$). The solvent was removed *in vacuo* and the residue distilled (air condenser) to give the aza-crown (4) (2.3 g, 85%), bp 72—74°C/0.01 mm Hg, mp 59—60°C, $t_R$ 7.1. Sublimation at 60°C/0.01 mm Hg gave needles of unchanged mp.

| | | | |
|---|---|---|---|
| $C_8H_{17}NO_3$ (175.2) requires: | C, 54.83; | H, 9.78; | N, 7.99% |
| Found: | C, 54.72; | H, 9.58; | N, 8.03%. |

$\gamma_{max}$ (Nujol mull) 3300 (NH),

δ (*H*) 2.70 (5H, symm. m with t structure, J 5, $OCH_2C\underline{H}_2NH$*) and 3.44—3.72 (12H, unsymm. m. $OC\underline{H}_2$) (*exchanges with $D_2O$, after which the m integrates for 4H),

δ (*C*) 48.73 (N—$\underline{C}$), 69.16, 69.81 and 71.11 (O—$\underline{C}$),

*m/e* 175 ($M^+$, 0.9) and 57 (100); $(M+1)^+$ (isobutane) 176.

Nujol is a Trade Mark.

Example 1

1,2-Bis(1-aza-4,7,10-trioxa-1-cyclododecanyl)ethane (5)

A solution of redistilled oxalyl chloride (0.26 g, 2.04 mmol) in dry, ethanol-free dichloromethane (about 8 ml, aliquot from standard solution) was added dropwise *via* a syringe to a stirred solution of aza-12-crown-4 (4) (0.71 g, 4.06 mmol) and triethylamine (1.5 g) in pure dichloromethane (30 ml). The clear solution was concentrated *in vacuo* to give a crystalline residue which was dissolved in dry tetrahydrofuran (100 ml). The stirred solution was cooled to 0°C and lithium aluminium hydride (1.0 g) was added. After stirring at room temperature for 18 hours, excess metal hydride was destroyed by the careful addition of a mixture of water (10 ml) and tetrahydrofuran (50 ml) with ice-cooling. Stirring was continued for 30 minutes before filtering (washing the residue with tetrahydrofuran) and concentrating the filtrate *in vacuo*. Distillation of the residue (the $^1H$ NMR spectrum of which was identical to that quoted below) at 0.05 mm Hg through a short path, with an air bath temperature rising to 200°C, gave the pure "headphones" (5) (0.65 g, 85%) as a colourless viscous oil, slowly solidifying on storage at 0°C, δ (*H*) 2.59 (4H, s, $NC\underline{H}_2C\underline{H}_2N$), 2.66 (8H, t, J 5, $OCH_2C\underline{H}_2N$), 3.56 (8H, t, J 5, $OC\underline{H}_2CH_2N$) and 3.59 (16H, s, $OC\underline{H}_2C\underline{H}_2O$), δ (*C*) 55.98 (N—$\underline{C}$ (bridge)), 56.50 (N—C (ring)), 71.09, 71.09 and 71.87 (O—C), *m/e* 376 ($M^+$, 2) and 188 (100).

Example 2

1,2-Bis(1-aza-4,7,10-trioxa-1-cyclododecanyl)ethane (5)

Ethylene ditosylate was prepared using the general procedure for ditosylating oligoethylene glycols of Kristiansen and Dale. Recrystallisation from absolute ethanol (15 ml/g) gave needles, mp 127—128°C, δ (*H*) 2.38 (6H, s, $C\underline{H}_3$), 4.08 (4H, s, $OC\underline{H}_2$), 7.17 and 7.56 (two 4H, d, J 8, arom. $A_2X_2$ system). A stirred solution of aza-12-crown-4 (4) (1.95 g, 11.1 mmol) and ethylene ditosylate (2.16 g, 5.8 mmol) in dry acetonitrile (60 ml) containing suspended powdered anhydrous sodium carbonate (2.5 g) was heated under reflux under an atmosphere of nitrogen for 48 hours. The suspension was cooled and filtered, and the filtrate concentrated *in vacuo* to give the crystalline 1:1 complex of the "headphones" (5) with sodium p-toluenesulphonate containing a trace of unreacted ethylene ditosylate.

(Complex: δ 2.26 (3H, s, $C\underline{H}_3Ar$), 2.30—2.84 (complex m*, $OCH_2C\underline{H}_2N$), 2.40 (s*, $NC\underline{H}_2C\underline{H}_2N$), 3.25—3.90 (24H, m, $OC\underline{H}_2C\underline{H}_2O$), 6.94 and 7.64 (two 2H, d, J 8, arom. $A_2X_2$ system) (* these signals together integrate for 12 H).

The free liquid was distilled from the crude complex at 0.01 mm through a short path with an air bath temperature rising slowly to 250°C. Redistillation using the same technique have the pure "headphones" (5) (1.75 g, 84%), having physical properties identical to those described in Example 1.

Example 3

1,4,10,13-tetraaza-7,16,21,26-tetraoxatricyclo[11,5,5,$5^{4,10}$]octacosane

The tetrahydropyranyl ether of 2-(2-chloroethoxy)ethanol was reacted with tosylamide in DMF in the presence of suspended NaH, to give the bis-tetrahydropyranyl ether. Treatment with p-toluenesulfonic acid in methanol yielded the free diol which was converted to the dimesylate with methanesulfonyl chloride in pyridine. The ether, diol and dimesylate were oils whose purity was checked by n.m.r., m.s. and t.l.c. Adapting the above procedure for preparing mono-aza-12-crown-4 compound by double alkylation of primary amines with 1,11-diiodo-3,6,9-trioxaundecane, the dimesylate was heated with benzylamine in refluxing acetonitrile containing NaI and suspended $Na_2CO_3$ for 48 hours, to give 1-benzyl-7-tosyl-1,7-diaza-4,10-dioxacyclododecane, m.p. 95°C (60%). $LiAlH_4$ reduction in THF yielded the mono-substituted diaza-crown compound 1-benzyl-1,7-diaza-4,10-dioxacyclododecane, m.p. 48—50°C (91%).

The first bridge was established by refluxing an acetonitrile solution containing the mono-substituted diaza-crown and ethylene ditosylate, in the presence of suspended $Na_2CO_3$, for 48 hours. The crude product was an oil which solidified on standing and had the correct spectral data for the dibenzylated bis-crown (*m/e* 554). This product was directly dibenzylated by hydrogenolysis over Pd/C to yield the unblocked bis-crown, i.e. 1,2-bis(1,7-diaza-4,10-dioxa-1-cyclododecanyl)ethane, b.p. 240°C/0.05 mm Hg, m.p. 98—100°C, *m/e* 374, in a yield of 85% based on the mono-substituted diaza-crown.

The closing of the second bridge with ethylene ditosylate presented difficulties. In the absence of a template cation, polymeric material only was formed, while the presence of sodium salts (carbonate or tosylate) led only to complexing of the unblocked bis-crown and no reaction. In view of the lower coordination number of lithium, it was proposed that this cation might leave the two weakest donor atoms (>NH) free to undergo reaction, but still be sufficiently strongly sandwiched, intra-molecularly, to exert a template effect. In fact, reaction took place when a solution of the unblocked bis-crown, dried lithium tosylate, ethylene ditosylate and ethyldiisopropylamine in acetonitrile was heated in a sealed tube at 105°C for 4 days. Contact with a brine solution during work-up led to isolation of a Na-rosylate complex ("cryptate") of the cage ligand title product ("cryptand") as pale yellow needles (81%).

The product's chemical ionisation mass spectrum (isobutane) corresponded to the dissociated protonated ligand (*m/e* 401, $(M+1)^+$), while the field desorption mass spectrum corresponded to the cation complex (*m/e* 423, $(M+23)^+$). The $^{13}C$ n.m.r. spectrum in acetonitrile showed three lines for the ligand in the expected intensity ratio 8:4:8, with shifts very close to those observed for chemically-comparable carbon atoms of the complexed singly-bridged bis-crown ligand (Example 1). These shifts did not change after passage through a cation exchange resin or a mixed-bed ion-exchange resin (elution with water, then with aqueous dimethylamine). Pyrolysis *in vacuo* (150—220°C/0.05 mm Hg) liberated the ligand, m.p. 160°C, which had all three $^{13}C$ lines moved by about 4 ppm downfield, similar to what is observed for the related ligands (see the Table). This means that the most likely conformation of the complexed ligand, which undoubtedly has all nitrogen lone pairs pointing into the cavity, is not retained by the free ligand. Space-filling molecular models do not allow much conformational choice for this tricyclic molecule; they suggest that two of the nitrogen line pairs are turned out and that both 12-membered rings adopt a conformation with a reduced number of upfield γ-interactions.

When the free ligand is contacted with a salt solution containing Li, Na and K cations and Br and $BF_4$ anions, a complex with $NaBF_4$ crystallises (m.p. >310°C). The X-ray structure determination confirms the expected cubic arrangement of the eight donor atoms.

TABLE

| Compound | $^{13}C$ nmr shifts ring $OCH_2$ | ring $NCH_2$ | bridge $NCH_2$ |
|---|---|---|---|
| free (5) | 65.2 | 52.4 | 52.4 |
| $Na^+$-complexed-(5) | 71.1 | 56.5 | 56.0 |
| free (6) | 65.8 | 52.2 | 51.8 |
| $Na^+$-complexed-(6) | 69.3 | 56.2 | 55.9 |

**Claims**

1. 1,2-Bis(1-aza-4,7,10-trioxa-1-cyclododecanyl)ethane.

2. 1,4,10,13-Tetraaza-7,16,21,26-tetraoxatricyclo[11,5,5,$5^{4,10}$]octacosane.

3. The use of the compound of claim 1 or claim 2, as a complexing agent.

4. A process for preparing the compound of claim 1, which comprises reacting 2-(1-aza-4,7,10-trioxa-1-cyclododecanyl)ethylamine with bis[2-(2-iodoethoxy)ethyl] ether.

5. A process for preparing the compound of claim 1, which comprises reacting 1-aza-4,7,10-trioxa-1-cyclododecane with a coupling reagent which provides an ethylene bridge.

6. A process for preparing the compound of claim 2, which comprises reacting a compound of the formula

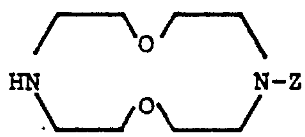

in which Z represents a blocking group, with a coupling reagent which provides an ethylene bridge; removing the blocking groups to give 1,2-bis(1,7-diaza-4,10-dioxa-1-cyclododecanyl)ethane; and reacting that unblocked, bridged compound with a coupling reagent which provides an ethylene bridge, in the presence of a template cation.

7. A process according to claim 6, in which the template cation is lithium.

8. A process according to any of claims 5 to 7, in which the or each coupling reagent is ethylene ditosylate.

**Patentansprüche**

1. 1,2-Bis(1-aza-4,7,10-trioxa-1-cyclododecanyl)ethan.

2. 1,4,10,13-Tetraaza-7,16,21,26-tetraoxatricyclo[11,5,5,$5^{4,10}$]octacosan.

3. Verwendung der Verbindung nach Anspruch 1 oder Anspruch 2 als Komplexierungsmittel.

4. Verfharen zur Herstellung der Verbindung nach Anspruch 1, welches Verfahren ein Umsetzen von 2-(1-Aza-4,7,10-trioxa-1-cyclododecanyl)ethylamin mit Bis[2-(2-jodoethoxy)ethyl]ether umfaßt.

5. Verfahren zur Herstellung der Verbindung von Anspruch 1, welches Verfahren ein Umsetzen von 1-Aza-4,7,10-trioxa-1-cyclododecan mit einem eine Ethylenbrücke liefernden Kupplungsmittel umfaßt.

6. Verfahren zur Herstellung der Verbindung von Anspruch 2, welches Verfahren ein Umsetzen einer Verbindung der Formel

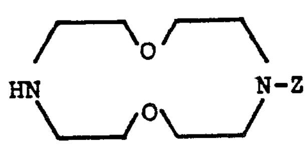

worin Z eine blockierende Gruppe darstellt, mit einem eine Ethylenbrücke liefernden Kupplungsmittel; ein Entfernen der blockierenden Gruppen zur Ausbildung von 1,2-Bis(1,7-diaza-4,10-dioxa-1-cyclododecanyl)ethan; und ein Umsetzen dieser umblockierten, verbrückten Verbindung mit einem eine Ethylenbrücke liefernden Kupplungsmittel in Gegenwart eines Modellkations umfaßt.

7. Verfahren nach Anspruch 6, worin das Modellkation Lithium ist.

8. Verfahren nach einem der Ansprüche 5 bis 7, in welchem das oder jedes Kupplungsmittel Ethylenditosylat ist.

**Revendications**

1. Le 1,2-Bis(1-aza-4,7,10-trioxa-1-cyclododécanyl)éthane.

2. Le 1,4,10,13-tétraaza-7,16,21,26-tétraoxatricyclo[11,5,5,$5^{4,10}$]octacosane.

3. L'utilisation du composé selon la revendication 1 ou la revendication 2 comme agent complexant.

4. Procédé pour la préparation du composé selon la revendication 1, qui consiste à faire réagir la 2-(1-aza-4,7,10-trioxa-1-cyclododécanyl)éthylamine avec l'éthebis[2-(2-iodoéthoxy)-éthylique].

5. Procédé pour la préparation du composé selon la revendication 1, qui consiste à faire réagir le 1-aza-4,7,10-trioxa-1-cyclododécane avec un agent de couplage qui fournit un pont éthylène.

6. Procédé pour la préparation du composé selon la revendication 2, qui consiste à faire réagir un composé de formule

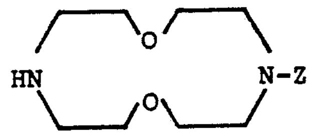

dans laquelle Z représente un groupe bloquant avec un réactif de couplage qui fournit un pont éthylène; à éliminer les groupes bloquants pour donner le 1,2-bis(1,7-diaza-4,10-dioxa-1-cyclododécanyl)éthane; et à faire réagir ce composé ponté non bloqué avec un réactif de couplage qui fournit un pont éthylène, en présence d'un cation calibré.

7. Procédé selon la revendication 6, dans lequel le cation calibré est le lithium.

8. Procédé selon l'une quelconque des revendications 5 à 7, dans lequel le ou chaque réactif de couplage est le ditosylate d'éthylène.